# EUROPEAN PATENT SPECIFICATION

(11) **EP 4 061 290 B1**
(45) Date of publication and mention of the grant of the patent: **01.07.2026**
(21) Application number: 20897602.7
(22) Date of filing: 12.11.2020
(51) Int. Cl.: A61F 2/78, A61F 2/80, A61L 27/02, A61L 27/16, B29C 70/78, C08K 5/01, C08L 53/02

(54) **LOW EXTENSIBILITY STRIPS FOR PROSTHETIC AND ORTHOTIC APPLICATIONS**
STREIFEN MIT NIEDRIGER DEHNBARKEIT FÜR PROTHETISCHE UND ORTHETISCHE ANWENDUNGEN
BANDES À FAIBLE EXTENSIBILITÉ POUR DES APPLICATIONS PROTHÉTIQUES ET ORTHÉTIQUE

(30) Priority: 21.11.2019 US 201962938483 P; 02.12.2019 US 201962942388 P; 11.11.2020 US 202017094885
(43) Date of publication of application: 28.09.2022
(73) Proprietor: Alps South Europe s.r.o, 32600 Plzen (CZ); Laghi, Aldo, Pinellas Park, FL 33782 (US)
(72) Inventor: LAGHI, Aldo, Pinellas Park, FL 33782 (US); VINT, Nathaniel, PalmHarbor, FL 34684 (US)
(74) Representative: Franks & Co Limited
(86) International application number: PCT/US2020/060091
(87) International publication number: WO 2021/113045

(56) References cited:
- WO-A1-02/091960
- IT-A1- MI20 111 636
- US-A- 5 263 923
- US-A1- 2002 123 709
- US-A1- 2003 181 989
- US-A1- 2005 101 693
- US-A1- 2006 111 792
- US-A1- 2016 338 858
- US-A1- 2016 338 858
- US-B1- 6 231 617
- US-B1- 6 475 174
- US-B1- 6 592 539
- US-B1- 9 308 111
- US-B2- 6 666 838

## Description

### BACKGROUND OF THE INVENTION

### Field of the Invention

The described invention relates to strips to prevent the elongation of prosthetics and orthotics in certain directions. Specifically, the described invention relates to low extensibility strips which reduce elongation in the longitudinal direction when applied to prosthetics and orthotics.

### Description of the Background Art

Silicone liners have been used since the 1980s in the prosthetic industry such as those described in U.S. Pat. No. 4,923,474 granted to Klasson and Kristinsson. Other examples of such liners include U.S. Pat. No. 5,728,168 to Laghi et al., U.S. Pat. No. 5,830,237 granted to Kania, U.S. Patent No. 5,507,834 to Laghi et al., U.S. Patent No. 5,443,525 to Laghi et al., and U.S. Patent No. 5,728,168 to Laghi et al. Gel and urethane liners have also been used for prosthetic and orthotic purposes and, for the most part, have a fabric covering. The fabric covering is used to reinforce the underlying material (silicone, gel, urethane) and allows for extensibility to make it easier to don and doff the liner by rolling it on and off the residual limb.

However, there exists a need for liners with limited extensibility in localized regions of the residual limb and greater extensibility in other regions of the residual limb. Liners such as those described in U.S. Pat. No. 9,216,099 to Laghi, U.S. Pat. No. 9,364,347, U.S. Patent 8,394,150, U.S. Pat. No. 8,852,291, U.S. Pat. No. 8,246,694, U.S. Pat. No. 8,808,294, U.S. Pat. No. 8,226,732, U.S. Pat. No. 8,357,206, U.S. Pat. No. 6,764,631, U.S. Pat. No. 6,544,292, U.S. Pat. No. 6,454,812, U.S. Pat. No. 5,728,168 and U.S. Pat. No. 5,507,834 each describe liners which highlight such a need.

U.S. Patent No. 6,231,617 to Fay describes a liner which incorporates elongate arms of a strip or ribbon shape. However, a limitation of Fay is that the arms have to radiate from the distal attachment plate or mounted to a peripheral edge of the distal attachment plate. The present invention allows for the use of low extensibility strips anywhere on or in the prosthetic/orthotic without being limited to a certain location on the device.

Generally, the distal end of locking liners require the fabric to be less extensible in the longitudinal direction or that a polymer or other solid implement be used in order to reduce pistoning of the prosthetic implement during ambulation or movement. Other regions may benefit from reduced extensibility of the fabric cover such as in the case the above the knee amputees who may exhibits unusually fleshy residual limbs.

US 2003/181989 A1 (Eberle, John) discloses a prosthetic liner having a closed distal end and an open proximal end having an elastomer layer and the fabric layer with an external face and a strip of low extensibility material fixed to the external face of the fabric layer.

WO 02/091960 A1 (Fay, John) discloses a prosthetic liner having a closed distal end and that an open proximal end having an elastomer layer and a nylon layer.

US 2005/101693 A1 (Arbogast, Robert) discloses a locking liner for a prosthetic limb aimed at preventing pistoning of the residual limb within the liner, having a gel insert, a two-way stretch material, and a non-stretch material extending up to a proximal upper end of the liner.

The present invention overcomes the aforementioned inadequacies of the prior art and current need in the industry by using low extensibility fabric adhered externally to the fabric cover of the liner or integrally formed with the liner internally.

Therefore, it is an object of this invention to provide an improvement which overcomes the aforementioned inadequacies of the prior art devices and provides an improvement which is a significant contribution to the advancement of the liner art.

Another object of the invention is to provide strips or other shapes of low extensibility material which can be applied to the exterior of fabric liners. [00014] Another object of the invention is to provide low extensibility material which, when applied to fabric liners, reduce extensibility in the longitudinal direction while maintaining extensibility in the transversal direction.

Another object of the invention is provide a fast and inexpensive way to reduce the extensibility of fabric liners in desired regions.

Another object of the invention is to provide a customizable solution for patient comfort such that a prosthetist can adhere appropriate shapes in appropriate locations in order to support or contain a region or segment of the residual limb as required by patient clinical conditions.

Another object of the invention is to contain the dynamic deformation of the residual limb during ambulation.

Another object of the invention is to provide a low extensibility material which can be cut into customer or standard shapes.

The foregoing has outlined some of the pertinent objects of the invention. These objects should be construed to be merely illustrative of some of the more prominent features and applications of the intended invention. Many other beneficial results can be attained by applying the disclosed invention in a different manner or modifying the invention within the scope of the disclosure. Accordingly, other objects and a fuller understanding of the invention may be had by referring to the summary of the invention and the detailed description of the preferred embodiment in addition to the scope of the invention defined by the claims taken in conjunction with the accompanying drawings.

### SUMMARY OF THE INVENTION

The present invention relates generally to low extensibility strips for use with prosthetic/orthotic devices. Specifically, the present invention relates to low extensibility material for use in prosthetics and orthotics which is adhered to the fabric portion of a prosthetic liner or orthotic liner either internally or externally and limits the longitudinal movement while allowing for transversal movement. The customizable nature of the invention allows for shaping and personalization depending on the needs of the user. According to one aspect of the invention there is provided an extensible orthotic device or prosthetic device, said device comprising:
a liner comprising an elastomer layer and a fabric layer;
said fabric layer (24) having a proximal end (28) and a distal end;
said elastomer layer being adhered to said fabric layer;
characterised by:
   at least one strip (26) of an extensible material (10);
   a gel, silicon or urethane material being molded over said fabric and said extensible material;
   said at least one strip of extensible material having a central aperture and a bulged mid region;
   said extensible material (26) being adhered to said fabric layer thereby creating a customised reinforced reduced stretch area;
   said extensible material being selected from the set silk, fiberglass cloth, carbon fiber, and a thermoplastics material;
   said at least one strip of extensible material (26) being adhered to said fabric layer within 15.24cm (6 inches) of said proximal end;
   said extensible material having a width between 1.27cm (0.5 inches) and 10.16cm (4 inches);
   use of the low extensibility material (10) limits the longitudinal stretch of the fabric layer (12) whilst maintaining its transversal stretch such that:
      the reinforced reduced stretch area has a reduced longitudinal stretch of more than 50% reduction as compared to a non-reinforced area of said liner (12) not having said extensible material; and
      the reinforced reduced stretch area has a transversal stretch which is the same as the transversal stretch of the unreinforced fabric layer;
      wherein the maximum transversal stretch of the reinforced reduced stretch area at failure is between 54.81% and 69.23% of its unstretched state.

The foregoing has outlined rather broadly the more pertinent and important features of the present invention in order that the detailed description of the invention that follows may be better understood so that the present contribution to the art can be more fully appreciated. Additional features of the invention will be described hereinafter which form the subject of the claims of the invention.

### BRIEF DESCRIPTION OF THE DRAWINGS

For a more complete understanding of the present disclosure and its advantages, reference is now made to the following descriptions, taken in conjunction with the accompanying drawings, in which:
Fig. 1 is a cross-sectional view of a liner incorporating the low extensibility material internally;
Fig. 2 is a top view of the low extensibility material in one preferred shape;
Fig. 3 is a top view of the low extensibility material in a second preferred shape;
Fig. 4 is a top view of the low extensibility material in a third preferred shape and configuration;
Fig. 5 is a top view of the low extensibility material in a second preferred configuration;
Fig. 6 is a cross-sectional view of a liner with an alternative arrangement of low extensibility material placed either internally or externally;
Fig. 7 is a cross-sectional view with an alternative arrangement of low extensibility material placed either internally or externally;
Fig. 8 is a perspective view of a wrist orthotic with the low extensibility material adhered externally;
Fig. 9 is a perspective view of a back orthotic with the low extensibility material adhered externally;
Fig. 10A is a perspective view of an ankle orthotic with the low extensibility material adhered externally;
Fig. 10B is a cross-sectional view of the ankle orthotic shown in Fig. 10A;
Fig. 11 is a perspective view of a knee orthotic with the low extensibility material adhered externally; and
Fig. 12 is a perspective view of an elbow orthotic with the low extensibility material adhered externally.

Similar reference numerals refer to similar parts throughout the several views of the drawings.

### DETAILED DESCRIPTION OF THE PREFERRED EMBODIMENT

The following description is of the best mode presently contemplated for carrying out the invention. This description is not to be taken in a limiting sense, but is made merely for the purpose of describing one or more preferred embodiments of the invention. The scope of the invention should be determined with reference to the claims. One inch corresponds to 2.54 cm.

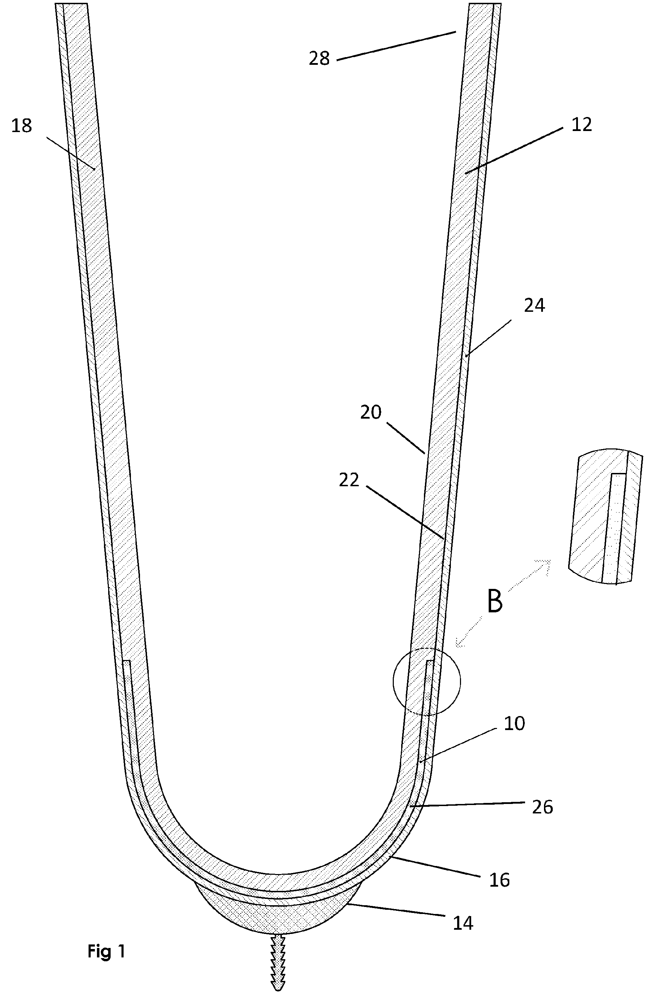

The present invention relates to a low extensible material 10 for use with prosthetic or orthotic devices. As shown in Fig. 1, this low extensibility material 10 can be used with a prosthetic liner 12 having a distal attachment plate 14 at a distal end 16. The low extensibility material 10 for use with a prosthetic or orthotic assembly preferably comprises a strip of material 26 preferably made of strips of silk, fiberglass cloth, and other non-elastic materials like carbon fiber and thermoplastics. The prosthetic liner 12 comprises an elastomer layer 18 having a limb face 20 and a fabric face 22 wherein the fabric face 22 of the elastomer layer 18 is adhered to the external fabric 24 of the prosthetic liner 12. In relation to the prosthetic liner 12, the low extensibility material 10 can be adhered to the fabric 24 either internally or externally. If internal placement is desired, the low extensibility material 10 is placed between the elastomer layer 18 and the fabric 24 in the desired region and adhered only to the fabric 24 as shown in the exploded view Part B. Preferably, the low extensibility material 10 is placed at the distal end 16 of the prosthetic liner 12 and extends towards the proximal end 28 of the prosthetic liner 12. The low extensibility material 10 preferably extends upward towards the proximal end 28 of the prosthetic liner 12, stopping at least an inch from the proximal end 12, when placed at the distal end 16. Similarly, the width of the low extensibility material 10 is between 0.5-4 inches such that it can be placed in a variety of locations and positions.

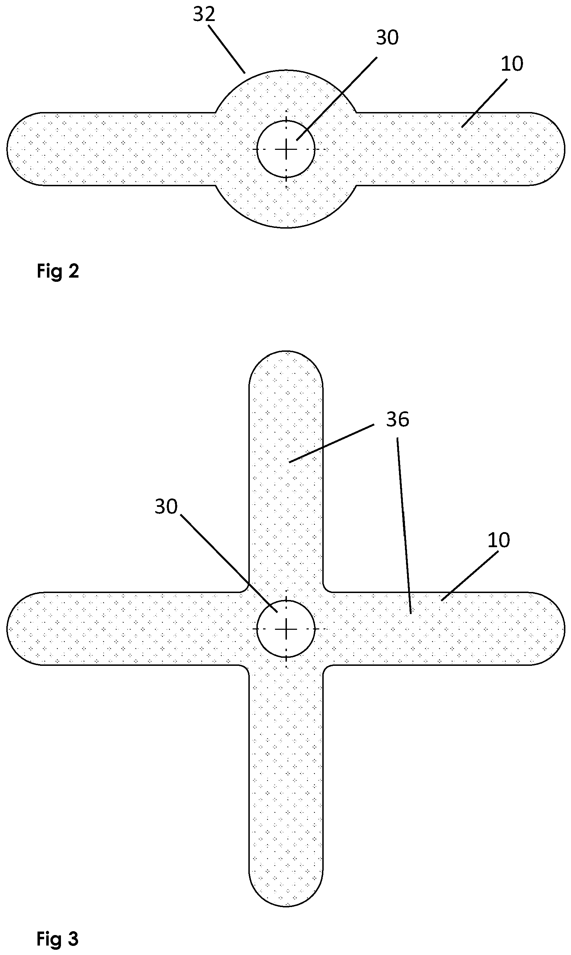

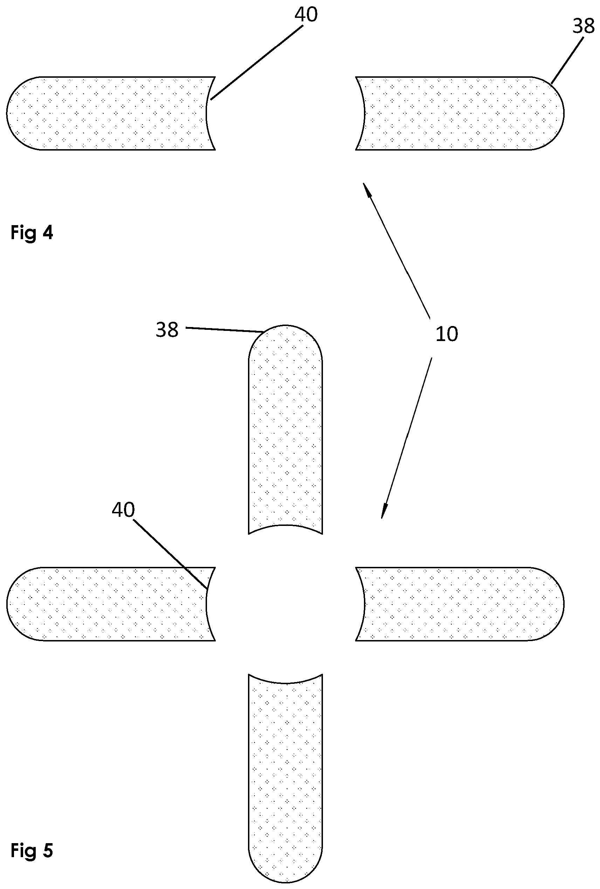

Figs. 2-7 show the low extensibility material 10 is a variety of configurations. Fig. 2 shows the low extensibility material 10 as a strip of material having a central aperture 30 with a bulged mid-region 32. The locking pin 34, as shown in Fig. 1, can be fed through the aperture 30 if the low extensibility material 10 is adhered externally to the fabric 24. As shown in Fig. 3, the low extensibility material 10 can also be configured to have multiple arms 36 extending radially outward from the central aperture 30. As shown in Figs. 4 and 5, the aperture 30 is optional. The low extensibility material 10 can be shaped such that a distal strip end 38 is convex while a proximal strip end 40 is concave. This allows for the low extensibility material 10 to be placed around the distal attachment plate 14 if desired or around the user's joint without impinging on joint movement.

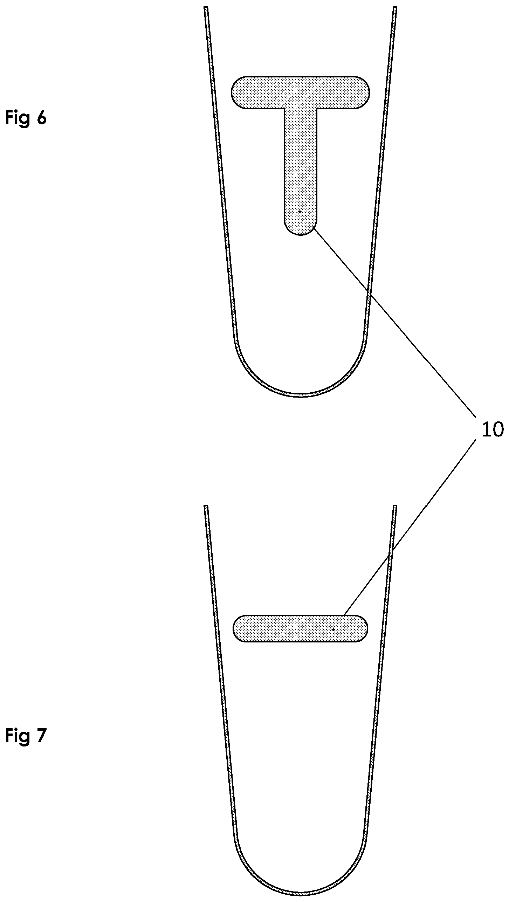

As seen in Figs. 6 and 7, the low extensibility material 10 can be placed in a variety of configurations according to the needs of the user. The T-shape and single strip configuration shown are not the only configurations that would be helpful to a user and any configuration determined by a medical professional to be medically helpful can be used.

Use of low extensibility material 10 as described herein has been tested and conclusively shows that use of the low extensibility material 10 limits the longitudinal stretch of the liner 12 while maintaining transversal stretch. In the first table, transversal stretch was tested:

| | Reinforced | | | | | Not Reinforced | | | |
|---|---|---|---|---|---|---|---|---|---|
| | before | after | change | % | | before | after | change | % |
| 1 | 13 | 21 | 8 | 61.53846 | 1 | 13 | 20.375 | 7.375 | 56.73077 |
| 2 | 13 | 22 | 9 | 69.23077 | 2 | 13 | 21.5 | 8.5 | 65.38462 |
| 3 | 13 | 20.125 | 7.125 | 54.80769 | 3 | 13 | 22 | 9 | 69.23077 |
| Average | | | | 61.85897 | Average | | | | 63.78205 |

As seen in the table above, "before" refers to the liner in a static state, meaning no weight was applied, and "after" refers to the liner at failure (i.e. until the strip tore). The number measured is circumference in inches. As can be seen from the test data, the transversal stretch was essentially the same whether the liner was reinforced with the low extensibility material 10 or not.

A similar test was performed testing the longitudinal stretch:

| | Reinforced | | | | | Not Reinforced | | | |
|---|---|---|---|---|---|---|---|---|---|
| | before | after | change | % | | before | after | change | % |
| 1 | 4.25 | 4.5 | 0.25 | 5.882353 | 1 | 4 | 4.75 | 0.75 | 18.75 |
| 2 | 4.25 | 4.5 | 0.25 | 5.882353 | 2 | 4.25 | 4.75 | 0.5 | 11.76471 |
| 3 | 4.5 | 4.75 | 0.25 | 5.555556 | 3 | 4.25 | 4.625 | 0.375 | 8.823529 |
| Average | | | | 5.77342 | Average | | | | 13.11275 |

As can be seen from this test, the longitudinal stretch was reduced by more than half when the low extensibility material 10 was used.

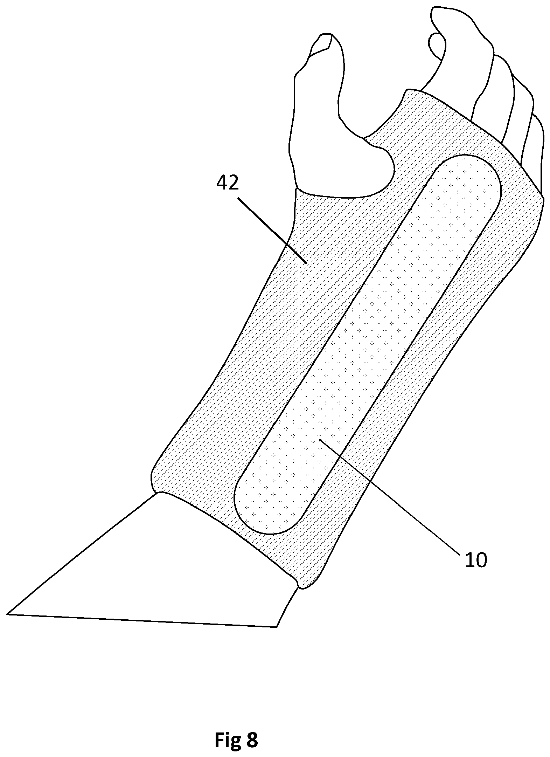

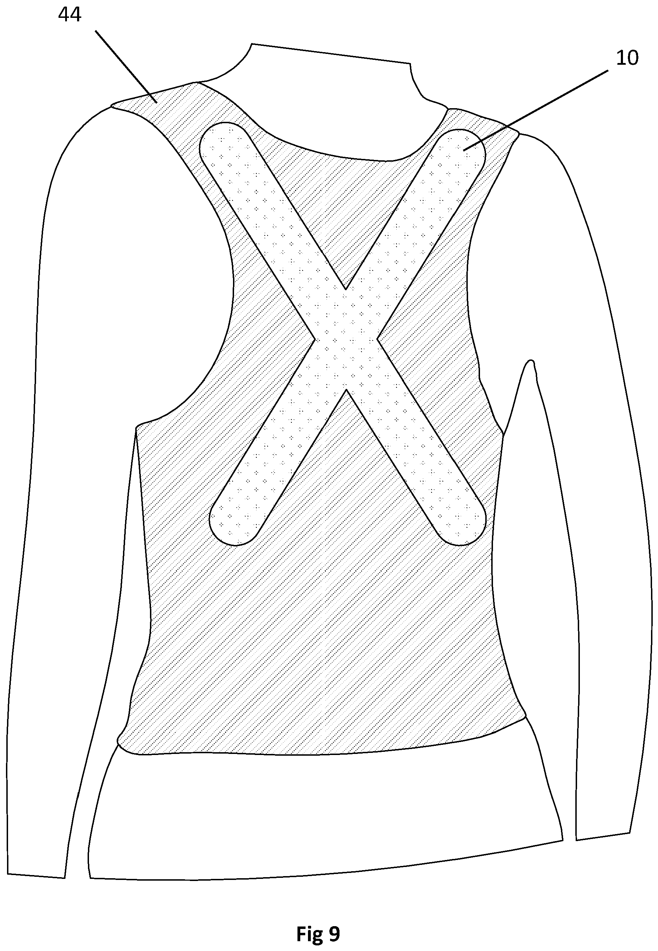

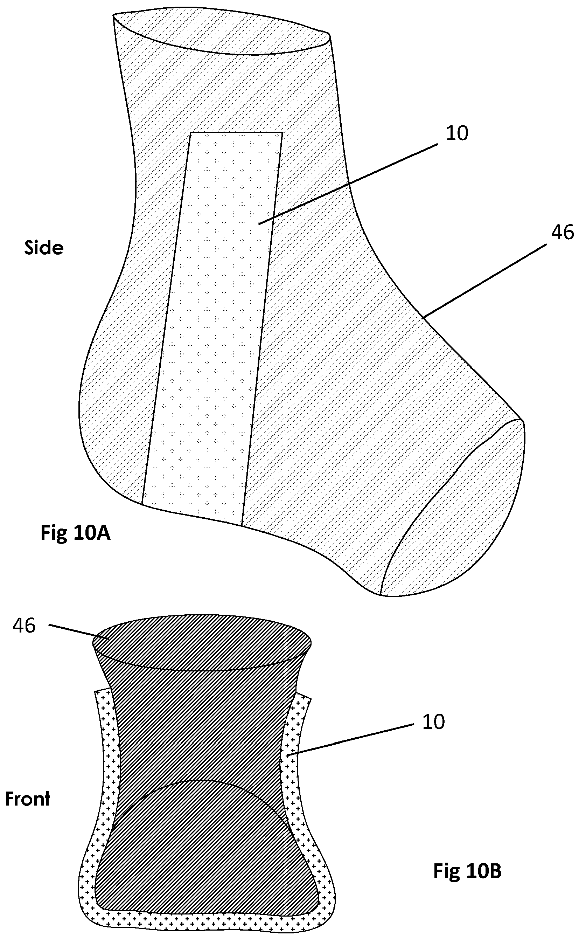

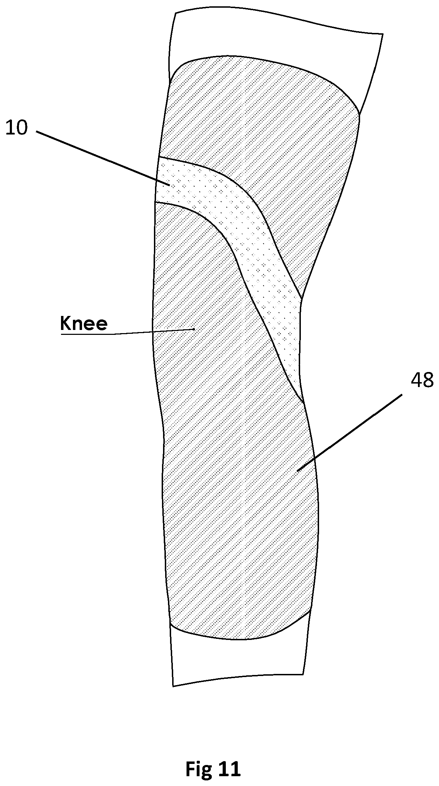

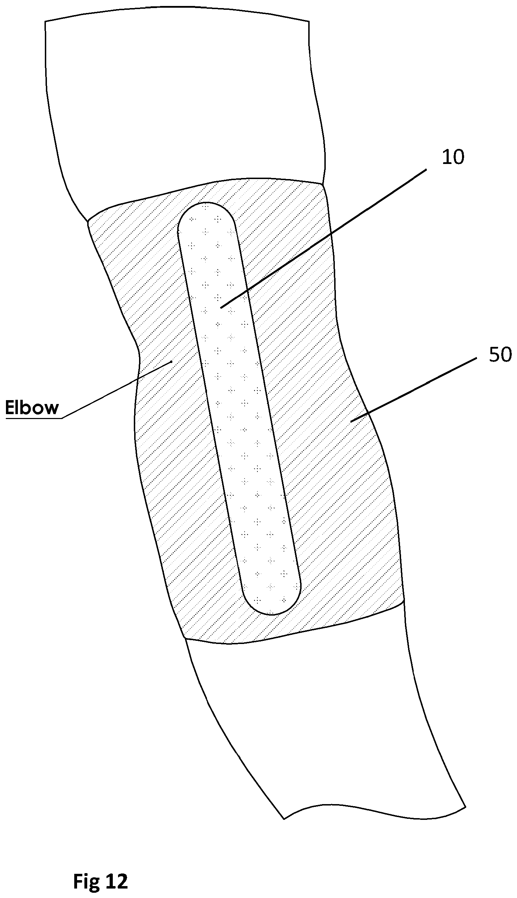

The low extensibility material 10 can also be used with orthotics which is useful to limit joint flexion both as a therapeutic aid in the case of injuries or as a means of injury prevention. As seen in Fig. 8, the low extensibility material 10 works with a wrist brace 42 and can be adhered externally or internally as described above. Similarly, as shown in Fig. 9, the low extensibility material 10 can be integrated with a back brace orthotic 44. Likewise, the low extensibility material 10 can be used with an ankle orthotic 46, as shown in Figs. 10A and 10B, where the low extensibility material 10 preferably has a thickness to prevent ankle movement. Fig. 11 depicts the use of the low extensibility material 10 with a knee orthotic 48 and Fig. 12 depicts the use of the low extensibility material 10 on an elbow orthotic 50. In all configurations when in use with an orthotic, the low extensibility material 10 can be in a strip or in a specific shape, as determined by an orthotic physician, and be adhered to the fabric 24 either internally or externally.

When the low extensibility material 10 is desired to be used internally, the low extensibility material 10 is first adhered to the fabric 24 using conventional means and then the combination is placed in a mold wherein the gel, silicon, or urethane is molded over, creating a composite. Alternatively, exterior use is performed by adhering the low extensibility material 10 onto the outside of the fabric 24 in the desired location.

## Claims

1. An extensible prosthetic device, said device comprising:
a liner comprising an elastomer layer (18) and a fabric layer (24);
said fabric layer (24) having a proximal end (28) and a distal end;
said elastomer layer being adhered to said fabric layer;
at least one strip (26) of an extensible material (10); **characterized by**
a gel, silicon or urethane material being molded over said fabric and said extensible material;
said at least one strip of extensible material having a central aperture and a bulged mid region;
said extensible material (26) being adhered to said fabric layer thereby creating a customised reinforced reduced stretch area;
said extensible material being selected from the set silk, fiberglass cloth, carbon fiber, and a thermoplastics material;
said at least one strip of extensible material (26) being adhered to said fabric layer within 15.24cm (6 inches) of said proximal end;
said extensible material having a width between 1.27cm (0.5 inches) and 10.16cm (4 inches); and
use of the extensible material (10) limits the longitudinal stretch of the fabric layer (12) whilst maintaining its transversal stretch such that:
the reinforced reduced stretch area has a reduced longitudinal stretch of more than 50% reduction as compared to a non-reinforced area of said liner (12) not having said extensible material; and
the reinforced reduced stretch area has a transversal stretch which is the same as the transversal stretch of the unreinforced fabric layer;
wherein the maximum transversal stretch of the reinforced reduced stretch area at failure is between 54.81% and 69.23% of its unstretched state.

2. An extensible prosthetic device according to claim 1, wherein:
said liner has a closed distal end and an open said proximal end;
said liner comprising a single piece of said low extensibility material (10) formed in between said elastomer layer (18) and said fabric layer (24).

3. An extensible prosthetic device according to claim 1, wherein:
said liner has a closed distal end and an open proximal end;
said fabric layer having an external face;
said liner comprising at least one strip of said extensible material affixed to said external face of the fabric layer.

4. An extensible prosthetic device according to Claim 3, wherein at least two said strips of extensible material are affixed to the external face of the fabric layer in a T-shape (10).

## Patentansprüche

1. Dehnbare prothetische Vorrichtung, wobei die Vorrichtung Folgendes umfasst:
eine Auskleidung, die eine Elastomerschicht (18) und eine Gewebeschicht (24) umfasst;
wobei die Gewebeschicht (24) ein proximales Ende (28) und ein distales Ende aufweist;
wobei die Elastomerschicht an der Gewebeschicht haftet;
zumindest einen Streifen (26) eines dehnbaren Materials (10);
**dadurch gekennzeichnet, dass**
ein Gel-, Silikon- oder Urethanmaterial über das Gewebe und das dehnbare Material geformt ist;
der zumindest eine Streifen aus dehnbarem Material eine zentrale Öffnung und einen gewölbten Mittelbereich aufweist;
das dehnbare Material (26) an der Gewebeschicht haftet, wodurch ein individuell verstärkter Bereich mit reduziertem Stretch erzeugt wird;
das dehnbare Material aus der ausgehärteten Seide, Glasfasertuch, Kohlenstofffaser und einem thermoplastischen Material ausgewählt ist;
der zumindest eine Streifen aus dehnbarem Material (26) an der Gewebeschicht innerhalb von 15,24 cm (6 Zoll) von dem proximalen Ende haftet;
das dehnbare Material eine Breite zwischen 1,27 cm (0,5 Zoll) und 10,16 cm (4 Zoll) aufweist; und
Verwendung des dehnbaren Materials (10) den Längsstretch der Gewebeschicht (12) begrenzt, während sein Querstretch beibehalten wird, sodass:
der verstärkte Bereich mit reduziertem Stretch einen reduzierten Längsstretch von mehr als 50 % Reduzierung verglichen mit einem nicht verstärkten Bereich der Auskleidung (12) aufweist, der das dehnbare Material nicht aufweist; und
der verstärkte Bereich mit reduziertem Stretch einen Querstretch aufweist, welcher der gleiche wie der Querstretch der unverstärkten Gewebeschicht ist;
wobei der maximale Querstretch des verstärkten Bereichs mit reduziertem Stretch bei Versagen zwischen 54,81 % und 69,23 % seines ungestretchten Zustands ist.

2. Dehnbare prothetische Vorrichtung nach Anspruch 1, wobei:
die Auskleidung ein geschlossenes distales Ende und ein offenes proximales Ende aufweist;
die Auskleidung ein einziges Stück des Materials mit niedriger Dehnbarkeit (10) umfasst, das zwischen der Elastomerschicht (18) und der Gewebeschicht (24) gebildet ist.

3. Dehnbare prothetische Vorrichtung nach Anspruch 1, wobei:
die Auskleidung ein geschlossenes distales Ende und ein offenes proximales Ende aufweist;
die Gewebeschicht eine Außenfläche aufweist;
die Auskleidung zumindest einen Streifen des dehnbaren Materials umfasst, der an der Außenfläche der Gewebeschicht befestigt ist.

4. Dehnbare prothetische Vorrichtung nach Anspruch 3, wobei zumindest zwei der Streifen aus dehnbarem Material an der Außenfläche der Gewebeschicht in einer T-Form (10) befestigt sind.

## Revendications

1. Dispositif prothétique extensible, ledit dispositif comprenant :
une doublure comprenant une couche d'élastomère (18) et une couche de tissu (24) ;
ladite couche de tissu (24) possédant une extrémité proximale (28) et une extrémité distale ;
ladite couche d'élastomère étant collée à ladite couche de tissu ;
au moins une bande (26) d'un matériau extensible (10) ;
**caractérisé en ce que**
un matériau de gel, de silicium ou d'uréthane étant moulé sur ledit tissu et ledit matériau extensible ;
ladite au moins une bande de matériau extensible présentant une ouverture centrale et une région médiane bombée ;
ledit matériau extensible (26) étant collé à ladite couche de tissu, créant ainsi une zone d'étirement réduite renforcée personnalisée ;
ledit matériau extensible étant choisi parmi la soie durcie, le tissu de fibre de verre, la fibre de carbone et un matériau thermoplastique ;
ladite au moins une bande de matériau extensible (26) étant collée à ladite couche de tissu à moins de 15,24 cm (6 pouces) de ladite extrémité proximale ;
ledit matériau extensible présentant une largeur comprise entre 1,27 cm (0,5 pouce) et 10,16 cm (4 pouces) ; et
l'utilisation du matériau extensible (10) limite l'étirement longitudinal de la couche de tissu (12) tout en maintenant son étirement transversal de manière à ce que :
la zone d'étirement réduite renforcée présente un étirement longitudinal réduit de plus de 50 % de réduction par rapport à la zone non renforcée de ladite doublure (12) ne comportant pas ledit matériau extensible ; et
la zone d'étirement réduite renforcée présente un étirement transversal qui est le même que l'étirement transversal de la couche de tissu non renforcé ;
ledit étirement transversal maximal de la zone d'étirement réduite renforcée à la rupture étant compris entre 54,81 % et 69,23 % de son état non étiré.

2. Dispositif prothétique extensible selon la revendication 1 :
ladite doublure présentant une extrémité distale fermée et une extrémité proximale ouverte ;
ladite doublure comprenant une seule pièce dudit matériau à faible extensibilité (10) formée entre ladite couche d'élastomère (18) et ladite couche de tissu (24).

3. Dispositif prothétique extensible selon la revendication 1 :
ledit revêtement présentant une extrémité distale fermée et une extrémité proximale ouverte ;
ladite couche de tissu possédant une face externe ;
ladite doublure comprenant au moins une bande dudit matériau extensible fixée à ladite face externe de la couche de tissu.

4. Dispositif prothétique extensible selon la revendication 3, au moins deux desdites bandes de matériau extensible étant fixées sur la face externe de la couche de tissu en forme de T (10).
